# NEUE EUROPÄISCHE PATENTSCHRIFT

(11) **EP 3 788 991 B2**
(45) Veröffentlichungstag und Bekanntmachung des Hinweises auf die Entscheidung über den Einspruch: **01.07.2026**
(45) Hinweis auf die Patenterteilung: 28.06.2023
(21) Anmeldenummer: 19195938.6
(22) Anmeldetag: 06.09.2019
(51) Int. Cl.: A61F 2/36

(54) **VERBINDUNGSANORDNUNG ZWEIER KOMPONENTEN**
CONNECTING ASSEMBLY OF TWO COMPONENTS
AGENCEMENT DE RACCORDEMENT DE DEUX COMPOSANTS

(43) Veröffentlichungstag der Anmeldung: 10.03.2021
(73) Patentinhaber: CeramTec GmbH, 73207 Plochingen (DE)
(72) Erfinder: JUSZCZYK, Mateusz Maria, 91235 Velden (DE); GEBERT DE UHLENBROCK, Anne, 91367 Weissenohe (DE)
(74) Vertreter: Maikowski & Ninnemann Patentanwälte Partnerschaft mbB

(56) Entgegenhaltungen:
- WO-A1-2015/084791
- WO-A1-2017/114521
- DE-A1- 4 405 447
- US-A1- 2014 121 713

## Beschreibung

Die Erfindung betrifft eine Verbindungsanordnung eines orthopädischen Systems zum Verbinden mehrerer Komponenten nach dem Oberbegriff des Anspruchs 1.

Ein Vorsatz einer ersten orthopädischen Komponente, ist mit einer Ausnehmung beispielsweise in Form einer Nut, einer Bohrung einer zweiten orthopädischen Komponente sicher verbunden. Die Verbindungsanordnung kann als formschlüssig, kraftschlüssig oder reibschlüssig oder einer Kombination derer ausgebildet sein. Der Vorsatz der ersten orthopädischen Komponente kann in Form eines Kegelstumpfes ausgebildet sein. Die Nut der zweiten orthopädischen Komponente kann eine gewölbte, konvexe Oberfläche aufweisen. Das Zusammenfügen zweier Komponenten wird durch das Vorhandensein einer gewölbten Oberfläche an einer Komponente erleichtert. Es erfolgt eine Selbstjustierung bzw. -ausrichtung der Komponenten zueinander. Beschädigungen werden vermieden. Beim Verbinden der ersten mit der zweiten orthopädischen Komponente entsteht dadurch ein orthopädisches System, welches eine sichere linienhafte Verbindung zwischen zwei Komponenten aufweist.

Der Stand der Technik umfasst verschiedene Offenbarungen von Verbindungsmitteln zweier Teile.

So ist es bekannt, zwei Teile mittels einer kegelförmigen Verbindung formschlüssig miteinander zu verbinden. Eine solche Verbindung, ein Morsekegel oder Morsekonus, ist die genormte Form eines Werkzeugkegels zum Spannen von Werkzeugen, z. B. großen Bohrern, Reibahlen und Spannfuttern in der Werkzeugaufnahme einer Werkzeugmaschine. Die Drehmomentübertragung vom Hohlkegel der angetriebenen Werkzeugspindel auf den darin klemmenden Schaft des Werkzeugs erfolgt reibschlüssig durch Haftreibung infolge der Selbsthemmung. Die Neigungswinkel der Morsekegel betragen etwa 1 °26' bis etwa 1°30' relativ zur Längsachse. Eine sichere kegelförmige Verbindung durch Haftreibung bedarf einer erforderlichen Längsausdehnung der Verbindung.

Passende Konusverbindungen werden in einer Vielzahl von orthopädischen Vorrichtungen verwendet. Zum Beispiel können modulare Femurimplantate einen Morsekegel mit gegebenenfalls anderen Neigungswinkel verwenden, um das proximale Ende eines Schafts an einem Kugelkopf zu befestigen. Bei modularen Femurimplantaten kann der distale Schaft ein kegelstumpfförmiges proximales Ende umfassen, das den männlichen Teil der Verbindung aufweist, wobei eine Ausnehmung des mit dem Schaft zu verbindenden Teils eine passende Innenkegelform aufweist.

So offenbart z.B. die US 2014/0121713 ein System zum Verbinden von orthopädischen Komponenten, wobei ein Vorsprung einer ersten Komponente in eine Öffnung einer zweiten Komponente eingeführt wird. Dabei ist der Vorsprung der ersten Komponente in verschiedene Bereiche eingeteilt, wobei der Umfang des Vorsprungs der ersten Komponente verformbare Oberflächenelemente aufweist. Der Querschnitt der Bereiche weist ein gerades verjüngendes Profil auf. In einer speziellen Ausführungsform ist offenbart, dass der Querschnitt des Vorsprungs eine Verjüngung nach außen aufweist.

Das sichere, positionsgenaue Verbinden zweier orthopädischer Komponenten mittels einer Konusverbindung erfordert eine entsprechende Längsausdehnung des Verbindungsbereichs. Diese erforderliche Länge ist bei verschiedenen Anwendungen z.B. im Bereich des Schultergelenks nicht vorhanden. Deshalb können dort nur Konusverbindungen mit geringer Längsausdehnung verwendet werden. Trotz höchster Sorgfalt bei der Implantation besteht die Gefahr, dass die Verbindungselemente nicht orthogonal ihrer Längsachsen sondern gekippt eingesetzt werden. Dies kann in Beschädigungen der Komponenten, sowie zu einer eingeschränkten Beweglichkeit des Gelenks führen. Dadurch entstehen häufig auch Entzündungen des im Bereich des Implantats umgebenden Gewebes was wiederum mit möglichen Schmerzen verbunden sein kann.

DE 44 05 447 A1 als nächstliegender Stand der Technik zeigt eine Verbindungsanordnung eines orthopädischen Systems zum Verbinden mehrerer Komponenten, wobei die Verbindungsanordnung eine erste Komponente und eine zweite Komponente umfasst, an denen jeweils wenigstens eine Kontaktfläche angeordnet ist, wobei die Kontaktfläche der zweiten Komponente gekrümmt geformt ist und die Kontaktfläche der ersten Komponente als Konus ausgebildet ist.

Konusverbindungen mit geringerer Längsausdehnung haben einen Nachteil.

Trotz höchster Sorgfalt bei der Implantation besteht die Gefahr, dass die Verbindungselemente nicht orthogonal ihrer Längsachsen, sondern gekippt eingesetzt werden. Dies kann zu Beschädigungen der Komponenten, sowie zu einer eingeschränkten Beweglichkeit des Gelenks führen. Dadurch entstehen häufig auch Entzündungen des im Bereich des Implantats umgebenden Gewebes was wiederum mit möglichen Schmerzen verbunden sein kann.

Ausgehend davon liegt der Erfindung die Aufgabe zugrunde, eine sichere, dauerhafte, lösbare und positionsgenaue Anordnung zur Verbindung zweier orthopädischer Komponenten zu gewährleisten.

Erfindungsgemäß wird diese Aufgabe durch eine Verbindungsanordnung mit den Merkmalen des Anspruchs 1 gelöst.

Diese erfindungsgemäße Verbindungsanordnung hat folgende Vorteile:
Die gekrümmt ausgebildete Oberfläche unterstützt die orthogonale Ausrichtung beider Komponenten. Nach dem korrekten Zusammenfügen entsteht immer eine linienförmige Berührung der beiden Komponenten, wobei die linienförmige Berührung in Bezug auf den Vorsprung bzw. die Ausnehmung in sich geschlossen sein kann, d. h. einen Kreis bildet, der den Vorsprung umgreift bzw. die Ausnehmung umrundet. Die linienförmige Berührung verläuft bevorzugt in einer Ebene, kann aber auch hiervon abweichen.

Ein zusätzlicher technischer Effekt liegt darin: Sollte beim Zusammenfügen beider Komponenten, diese in einem Winkel zueinander ausgerichtet sein, der von einer orthogonalen Ausrichtung abweicht, können sich beide Komponenten zunächst an zwei Punkten berühren. Diese Zweipunktberührung wird unter Anwendung einer entsprechenden Kraft in eine linienförmige Berührung überführt. Dadurch wird eine sichere Verbindung beider Komponenten erzeugt.

Ein orthopädisches System weist wenigstens zwei Komponenten und eine erfindungsgemäße Verbindungsanordnung auf.

Die Erfindung beschreibt auch ein orthopädisches System, welches eine Anordnung zur Verbindung von zwei Komponenten umfasst. Ein Vorsatz, ausgebildet in Form einer Feder oder eines Vorsprungs, einer ersten Komponente, ist mit einer Ausnehmung in Form einer Nut einer zweiten Komponente sicher verbunden. Die Verbindung kann als formschlüssig, kraftschlüssig oder reibschlüssig oder einer Kombination derer ausgebildet sein. Der an einem ersten Ende einer ersten Komponente gebildete Vorsatz ist erfindungsgemäß in Form eines Kegelstumpfes, dessen Durchmesser ausgehend vom distalen Ende zunimmt, ausgebildet. Bei der Durchmesserzunahme kann es sich um eine konstante Zunahme handeln. Der Umfang des Vorsatzes kann, abhängig von der Form und der Position, unterschiedliche Abmessungen aufweisen. Die gesamte Oberfläche des Vorsatzes oder Abschnitte oder Teilbereiche dieser, sind Teil der Verbindungsanordnung und bilden ein Verbindungsmittel zwischen einer ersten und einer zweiten Komponente. Im montierten, zusammengebauten Zustand zweier Komponenten, steht die Oberfläche oder Teilbereiche dieser, einer ersten Komponente in Wirkverbindung mit einer Oberfläche oder Teilbereiche dieser, einer zweiten Komponente. Die Wirkverbindung entsteht entlang eines linienförmigen Kontakts, einer Umfangslinie.

In einem anderen Ausführungsbeispiel kann der Vorsatz wenigstens zwei Bereiche umfassen. Dabei können diese Bereiche getrennt, in einem Abstand zueinander angeordnet sein. Der Querschnitt des ersten Bereichs, der am distalen Ende der ersten Komponente angeordnet ist, ist kleiner als der Durchmesser des Querschnitts des zweiten Bereichs, der vom distalen Ende beabstandet angeordnet ist. Ein Bereich muss konisch ausgebildet sein. Der Winkel des Konus des ersten Bereichs, kann von dem Winkel des Konus des zweiten Bereichs abweichen. Er kann größer oder kleiner sein. Beide Winkel der Konusse können auch die gleichen Werte aufweisen. Der erste Bereich kann als Einführhilfe ausgebildet sein und die Implantation der Komponente erleichtern. Der zweite Bereich kann als Teil der Verbindungsanordnung dienen und die ortsfeste, sichere Positionierung der Komponente sicherstellen. Dazu weist der zweite Bereich des Vorsatzes die Flächenbereiche auf, mittels derer die Wirkverbindung hergestellt werden kann.

Die Nut, ausgebildet an einem zweiten Ende der zweiten orthopädischen Komponente wird durch eine Umfangsfläche und eine Grundfläche begrenzt. Die Umfangsfläche ist als gewölbte, gekrümmte Oberfläche ausgebildet. Ausgehend vom distalen Ende der Nut kann der Querschnitt der Nut über einen ersten Bereich abnehmen, bis der kleinste Querschnitt erreicht ist. Dieser kleinste Querschnitt, der Scheitelpunkt, kann etwa im Bereich der Hälfte der Tiefe der Nut liegen. Ab dem Scheitelpunkt bleibt der Querschnitt bis zum Grund der Nut konstant. Dadurch entsteht die Krümmung, eine konvex geformte Oberfläche der Ausnehmung der Nut, der zweiten Komponente. Die Nut eines Ausführungsbeispiels gemäß dieser Beschreibung weist eine Krümmung und einen Scheitelpunkt auf. Der Scheitelpunkt kann in einem beliebigen Abstand zu der Grundfläche der Nut angeordnet sein.

Die Durchmesser der Ausnehmung der zweiten Komponente und des Vorsprungs der ersten Komponente sind so aufeinander abgestimmt, dass beim Zusammenfügen eine dauerhaft sichere Verbindung entsteht und dies unabhängig davon, an welcher Komponente die gekrümmte Oberfläche ausgebildet ist. Die gekrümmt ausgebildete Oberfläche unterstützt die orthogonale Ausrichtung beider Komponenten. Nach dem korrekten Zusammenfügen entsteht immer eine linienförmige Berührung der beiden Komponenten, wobei die linienförmige Berührung in Bezug auf den Vorsprung bzw. die Ausnehmung in sich geschlossen sein kann, d.h. einen Kreis bildet, der den Vorsprung umgreift bzw. die Ausnehmung umrundet. Die linienförmige Berührung verläuft bevorzugt in einer Ebene, kann aber auch hiervon abweichen.

Sollte beim Zusammenfügen beider Komponenten, diese in einem Winkel zueinander ausgerichtet sein, der von einer orthogonalen Ausrichtung abweicht, können sich beide Komponenten zunächst an zwei Punkten berühren. Diese Zweipunktberührung wird unter Anwendung einer entsprechenden Kraft in eine linienförmige Berührung überführt. Dadurch wird eine sichere Verbindung beider Komponenten erzeugt.

Erfindungsgemäß weist eine von zwei Komponenten, in dem Bereich, in dem die beiden Komponenten zusammengefügt werden, eine gekrümmte Oberfläche auf. Erfindungsgemäß umfasst die Verbindungsanordnung eine einzige gekrümmte Oberfläche. Überraschenderweise wurde festgestellt, dass eine einzige gekrümmte Oberfläche ausreicht um eine sichere Verbindung zweier Komponenten bereit zu stellen.

Sollen zwei Komponenten mittels eines Adapters dauerhaft aber lösbar zu einem orthopädischen System verbunden werden, so kann die Verbindungsanordnung zwischen Adapter und erster Komponente und/oder zwischen Adapter und zweiter Komponente angeordnet sein. Dabei können sowohl der Adapter wie auch die Komponenten eine gekrümmte Oberfläche aufweisen. Es ist auch möglich, dass der Adapter zwei gekrümmte Oberflächen aufweist. Mittels einer ersten gekrümmten Oberfläche wird eine Verbindung zwischen Adapter und erster Komponente und mittels einer zweiten gekrümmten Oberfläche die Verbindung zwischen Adapter und zweiter Komponente hergestellt. Es entsteht ein orthopädisches System mit wenigstens drei Komponenten und wenigstens zwei Verbindungsanordnungen.

Ein Vorteil der Erfindung ist, dass der Kontaktpunkt der beiden Komponenten genau definiert ist. Er lässt sich vor dem Zusammenbau der beiden Komponenten genau berechnen und bleibt sowohl nach korrektem Einbau als auch bei schwerer Belastung immer konstant. Somit bleibt die Spannungsverteilung bzw. das Spannungsverteilungsmuster konstant, was bei Standardkonusverbindungen nicht der Fall ist. Außerdem ist ein Einklemmen ausgeschlossen, speziell bei einem großen Verhältnis von Durchmesser zu Höhe der Verbindung.

**Bezugszeichenliste**

| | |
|---|---|
| 1 | Erste orthopädische Komponente |
| 2 | Zweite orthopädische Komponente |
| 4,4' | Verbindungsanordnung |
| 5 | Vorsatz, Vorsprung |
| 6 | Distales Ende von 5 |
| 7 | Erstes Ende von 1 |
| 8 | Adapter |
| 9 | Nut von 8 |
| 10 | Nut von 2 |
| 11 | Zweites Ende von 2 |
| 12 | Boden, Grundfläche von 10 |
| 14 | Mündung von 10 |
| 16 | Umfangsfläche, Oberfläche |
| 17 | Schaft |
| 18 | Kugelkopf |
| 19 | Stelle |
| 20 | Flachseite |
| 21 | Flächenabschnitt |
| 22 | Flächenabschnitt |
| 23 | gekrümmter Flächenabschnitt |
| 26 | Außenumfangsfläche von 8 |
| 27 | Kontaktfläche |
| 28 | Umfangsfläche, Oberfläche |
| 29 | Kontaktfläche |
| 30 | gekrümmter Flächenabschnitt |
| 31 | Ende von 30 |
| 32 | Hohlzylinder |
| 33 | Längsachse |
| 34 | Ring oder Torus |
| 35 | Konus |
| 36 | Kontaktpunkte |
| 37 | Längsachse |
| 38 | Spalt |
| T | Tiefe von 10 |
| D1 | Durchmesser |
| D2 | Durchmesser von 2 |
| Q | Querschnitt |
| K | Scheitelpunkt |
| KP | Kontaktpunkt |
| H | Höhe |
| M | Schnittpunkt von 16 mit der Mündung von 10 |
| A | Schnittpunkt von 16 mit 1 |
| R | Radius von 16 |
| α | Winkel |
| B | Winkel |
| X | Abstand |
| Y | Mittelachse |
| Z | Ausschnitt aus Figur 6 |
| AB | Entfernung von KP zu K |
| Y | Mittelachse |

Ein weiterer Vorteil der Erfindung ist, dass sich zwei Komponenten im Verlauf, d. h. während des Einbauprozesses automatisch orthogonal ausrichten. Zusätzlich können durch eine erfindungsgemäße Verbindungsanordnung, während des Einbauprozesses Beschädigungen vermieden werden. Aufgrund der Erhöhung des Durchmessers einer Ausnehmung, im Anschluss an den Scheitelpunkt der Ausnehmung einer zweiten Komponente (nicht-beansprucht), kann das distale Ende, bzw. eine Kante am distalen Ende einer ersten Komponente, nach erfolgter Zweipunktanlage die Ausnehmung der zweiten Komponente nicht beschädigen. Es ist genügend Freiraum vorhanden, sodass nach dem Zentrieren beider Komponenten Beschädigungen vermieden werden. Unter Zentrieren wird in diesem Zusammenhang das manuelle Anlegen bzw. Einführen einer kegelstumpfförmigen ersten Komponente in eine Ausnehmung einer zweiten Komponente, oder umgekehrt verstanden. Dabei handelt es sich um einen der ersten Schritte beim Zusammenfügen zweier Komponenten, es entsteht eine Zweipunktanlage. In diesem Prozessstadium ist eine orthogonale Ausrichtung beider Komponenten zueinander nicht erforderlich bzw. gewährleistet.

Nachfolgend wird die Erfindung anhand von Figuren erläutert. Dabei zeigt
- Figur 1:: einen Querschnitt der Verbindung zweier nicht-erfindungsgemäßer Komponenten, auszugsweise, teilweise geschnitten und in schematischer Darstellung,
- Figur 2: die Verbindungsstelle gemäß Figur 1 vergrößert, auszugsweise und in schematischer Darstellung,
- Figur 3: eine nicht-erfindungsgemäße Ausführungsform der Verbindungsstelle auszugsweise und in schematischer Darstellung,
- Figur 4:: einen Querschnitt einer alternativen Form einer nicht-erfindungsgemäßen Verbindung von drei Komponenten, auszugsweise und in schematischer Darstellung,
- Figur 5:: die Verbindungsstelle gemäß Figur 1 vergrößert, auszugsweise und in schematischer Darstellung,
- Figur 6: eine erfindungsgemäße Ausführungsform der Verbindungsstelle auszugsweise und in schematischer Darstellung,
- Figur 7: den Ausschnitt Z aus Figur 6 in vergrößerter Darstellung nicht maßstabsgetreu zur besseren Erkennbarkeit des Wesentlichen,
- Figur 8: einen Querschnitt einer alternativen Form einer nicht-erfindungsgemäßen Verbindung mit einem großen Konuswinkel und damit einer flachen Bauform,
- Figur 9: die Verbindungsstelle gemäß Figur 8 vergrößert, auszugsweise und in schematischer Darstellung,
- Figur 10: ein Beispiel eines 2-Punktkontaktes zwischen einem Konus und einem Ring und
- Figur 11: den Konus gemäß Figur 10 mit eingezeichnetem Kontaktspalt zum Ring.

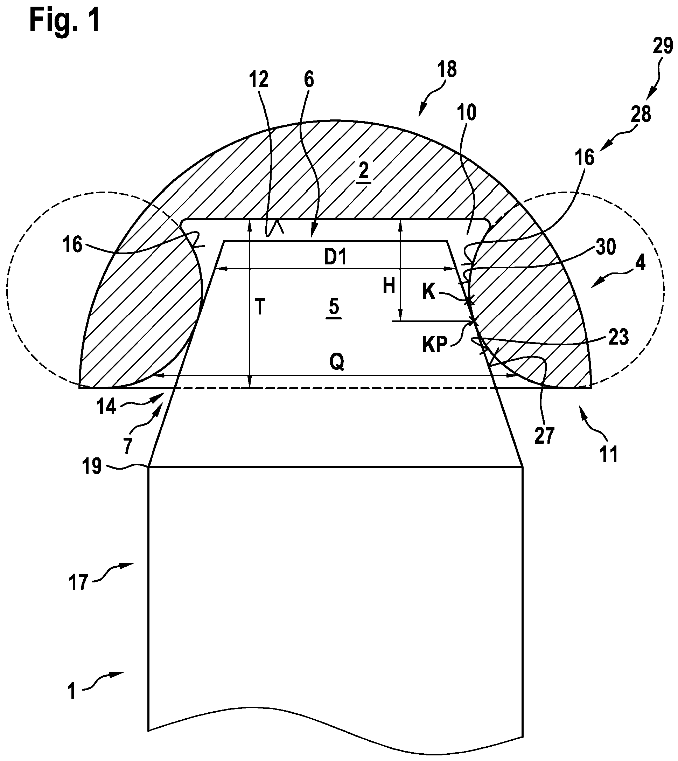

Figur 1 zeigt eine nicht-erfindungsgemäße Verbindungsanordnung 4 zweier orthopädischer Komponenten, hier eines Schafts 17 einer ersten Komponente 1 mit einem Kugelkopf 18 einer zweiten Komponente 2. Der in Figur 1 gezeigte Bereich des Schafts 17 weist an seinem ersten Ende 7 einen Vorsprung oder Vorsatz 5 auf. Ausgehend von einer Stelle 19 des Schafts 17 verringert sich der Durchmesser D1 des Vorsatzes 5 bis zu dessen distalen Ende 6 kontinuierlich. Der Vorsatz 5 ist im Ausführungsbeispiel gemäß Figur 1 in Form eines Kegelstumpfes ausgebildet. Der Kegelstumpf weist eine Umfangs- bzw. Kontaktfläche 27 auf. Beide Komponenten können Teil einer Hüftgelenkprothese, einer Schultergelenkprothese oder einer Finger- oder Fußgelenkprothese oder andere Gelenkprothesen sein.

Die zweite Komponente 2 weist eine Ausnehmung oder Nut 10 auf. Die Nut 10 wird durch ein Umfangsfläche 16 und einer Grundfläche beziehungsweise einem Boden 12 und gegenüber dem Boden von einer Mündung 14 begrenzt. Die Umfangsfläche 16 kann ganz oder teilweise als Kontaktfläche 29 ausgebildet sein. Die Kontaktfläche 29 kann in einem Abschnitt oder einem Bereich der Umfangsfläche 16 ausgebildet sein. Ausgehend vom zweiten Ende 11 der Komponente 2, von der Mündung 14 der Nut 10, ist die Umfangsfläche 16 der Nut 10 ganz oder teilweise gekrümmt ausgebildet. Durch die Krümmung entsteht eine konvex gekrümmte Oberfläche 28 die identisch mit der Kontaktfläche 29 und in Figur 1 identisch mit der Umfangsfläche 16 ist. Ausgehend von der Mündung 14 der Nut 10 nimmt der Betrag des Durchmessers beziehungsweise des Querschnitts Q der Nut 10 ab, bis zu einem Punkt, dem Scheitelpunkt K. Ab diesem Scheitelpunkt K nimmt der Betrag des Querschnitts Q wieder zu, bis in den Bereich in dem die Umfangsfläche 16 in die Grundfläche 12 der Nut 10 übergeht. Dadurch entsteht eine gekrümmt ausgebildete Oberfläche 28 der Nut 10, die einen Flächenabschnitt 23 der als Anstieg, einen Scheitelpunkt K und einen Flächenabschnitt 30, der als Abstieg ausgebildet ist, umfasst.

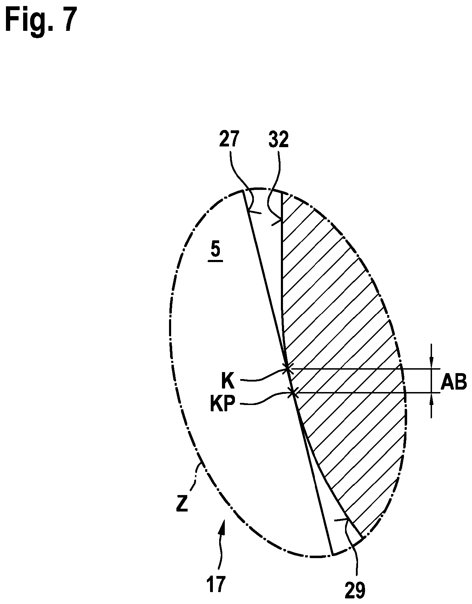

Beim Zusammenfügen des Schafts 17 der ersten Komponente 1 mit dem Kugelkopf 18 der zweiten Komponente 2 entsteht an einem Kontaktpunkt KP ein Kontakt beider Komponenten. Der Kontaktpunkt KP liegt in dieser Ausführungsform der Erfindung auf der Umfangsfläche 16 bzw. der Kontaktfläche 27 unterhalb des Scheitelpunkts K in Richtung zum zweiten Ende 11 bzw. zur Mündung 14. Die Entfernung AB (siehe Figur 7) des Kontaktpunktes KP vom Scheitelpunkt K richtet sich nach der Art des Kegelstumpfes der ersten Komponente und der Krümmung der Kontaktfläche 27.

Der Kontakt beider Komponenten befindet sich somit in dieser Ausführungsform unterhalb des Scheitelpunktes K in Richtung zur Öffnung hin. Der Kontaktpunkt KP, an dem die Wirkverbindung stattfindet, ist in einem Abstand zum Scheitelpunkt K auf der Kontaktfläche 27, 29 in Richtung zu der Mündung der zweiten Komponente 2 angeordnet. Nur wenn nicht erfindungsgemäß der Kegelstumpf kein solcher ist, sondern zum Beispiel ein Zylinder wäre, fällt Kontaktpunkt und Scheitelpunkt zusammen. Bei einem Kegelstumpf bzw. Konus mit einem sehr großen Winkel, liegt der Kontaktpunkt nahe der Öffnung der Nut 10.

Die Kontaktfläche 27 der Komponente 1 und die Kontaktfläche 29 der Komponente 2 stehen in Wirkverbindung. Durch die erfindungsgemäße Ausführung der gekrümmt ausgebildeten Kontaktfläche 29 bzw. Oberfläche 28 der Nut 10 und der Kontaktfläche 27 des Schafts 17 kann die Kontaktstelle beider Komponenten präzise bestimmt und festgelegt werden. Dies ist besonders der Fall, wenn die Kontaktfläche 27 eine mathematische Funktion bildet, wie erfindungsgemäß im Querschnitt ein Kreis. Ein Kreis ist besonders einfach zur Berechnung der Lage des Kontaktpunktes KP geeignet. Dies ist bei Komponenten, die aufgrund ihrer Anwendung nur geringe Ausdehnung in Längsrichtung haben können, von großem Vorteil. So ist eine sichere Verbindung beider Komponenten trotz geringer Tiefe T der Nut 10 erzielbar. Es können orthopädische Systeme mit sicheren Verbindungen zweier Komponenten im Bereich von Schultergelenken oder für Kleinkinder oder im veterinären Bereich zur Verfügung gestellt werden.

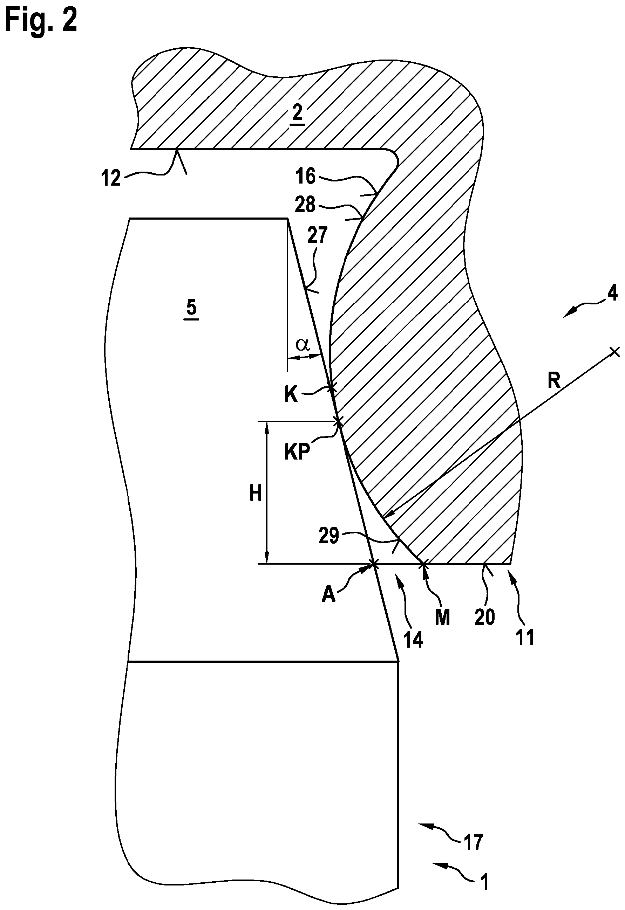

Figur 2 zeigt eine nicht-erfindungsgemäße Verbindungsanordnung 4 zweier Komponenten in vergrößerter Darstellung. Dabei ist ersichtlich, dass der Kontakt der ersten Komponente 1 mit der zweiten Komponente 2 sich auf einen schmalen Kontaktbereich konzentriert. Es entsteht ein Linienkontakt zwischen der Kontaktfläche 29 und der Kontaktfläche 27, am Kontaktpunkt KP.

Der Linienkontakt wird durch den Radius R der Kontaktfläche 29 bzw. Oberfläche 28 der Komponente 2 und dem Winkel α der Kontaktfläche 27 der Komponente 1 bestimmt. Durch entsprechende Veränderungen kann die Position des Kontaktpunkts KP bestimmt werden. Bei konstantem Radius R und kleinerem Betrag des Winkels α entfernt sich der Kontaktpunkt KP von der Mündung des zweiten Endes 11 der zweiten Komponente 2 hin zum Scheitelpunkt K, an dem der Durchmesser der Nut 10 am geringsten ist. Der Betrag von H, die Höhe in der der Kontaktpunkt KP von der Mündung 14 entfernt angeordnet ist, vergrößert sich. Wird dagegen bei gleichem Radius R der Winkel α größer reduziert sich der Abstand H des Kontaktpunktes KP von der Mündung 14 entsprechend.

Im Ausführungsbeispiel gemäß Figur 2 geht die gekrümmt ausgebildete Kontaktfläche 29, bzw. Oberfläche 28 an der Stelle M in das zweite Ende 11 der Komponente 2 über. Dieses zweite Ende 11 ist als Flachseite 20 ausgebildet. Es entsteht ein Schnittpunkt M zwischen der Kontaktfläche 29 der Nut 10 und der Flachseite 20. Der Schnittpunkt M ist der Punkt, der zugleich das Ende der Krümmung der Kontaktfläche 29 in Richtung des zweiten Endes 11 der Nut 10 festlegt. Nach dem Zusammenfügen der Komponenten 1 und 2 ist der Schnittpunkt M und ein Punkt A an der Kontaktfläche 27 am Schaft 17 der ersten Komponente 1, beabstandet zueinander angeordnet. Dabei liegt der Punkt A auf Höhe des Schnittpunktes M in horizontaler Verlängerung der Flachseite 20 in Richtung des Schafts 17 an der ersten Komponente 1. Eine erfindungsgemäße Verbindungsanordnung 4 weist zwischen dem Schnittpunkt M und dem Punkt A einen Abstand auf, der sich in Richtung zu dem Kontaktpunkt KP verringert, bis der Betrag des Abstands am Kontaktpunkt KP 0 ist.

Im Ausführungsbeispiel gemäß Figur 1 und 2 entspricht die Umfangsfläche 16 der Kontaktfläche 29 und der Oberfläche 28.

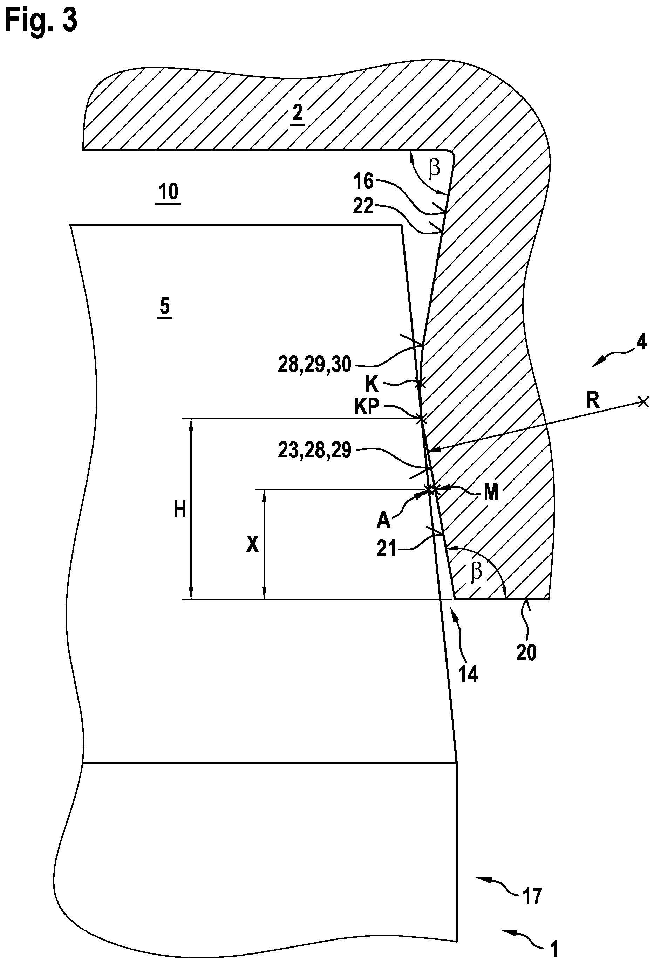

Figur 3 zeigt eine Ausführung einer nicht-erfindungsgemäßen Verbindung 4 zweier orthopädischer Komponenten 1 und 2. Die Umfangsfläche 16 der Nut 10 umfasst mehrere Abschnitte beziehungsweise Bereiche. Die Umfangsfläche 16 wird ausgehend von der Flachseite 20, der Mündung 14 der Nut 10 durch einen ebenen Flächenabschnitt 21 gebildet. Dieser Flächenabschnitt 21 ist in einem Winkel β zur Flachseite 20 angeordnet. An den Flächenabschnitt 21 schließt sich ein gekrümmter Abschnitt 23 an. Bezüglich der Krümmung dieses Flächenabschnitts 23 gilt vorstehende Beschreibung der Kontaktfläche 29. Der gekrümmte Abschnitt 23 reicht von einem Schnittpunkt M bis zum Scheitelpunkt K und ist als Anstieg ausgebildet. Dies bedeutet, der Durchmesser der Nut 10 reduziert sich im Bereich des Flächenabschnitts 23, ausgehend vom Schnittpunkt M bis zum Scheitelpunkt K. Am Übergang vom Flächenabschnitt 21 zu dem Flächenabschnitt 23 ist der Schnittpunkt M ausgebildet. Dieser Schnittpunkt M ist in einem Abstand X zu der Flachseite 20 angeordnet. Nach dem Zusammenfügen der beiden Komponenten 1 und 2 ist der Punkt A in horizontaler Verlängerung des Schnittpunkts M an der Kontaktfläche 27 am Schaft 17 der Komponente 1 und somit auch im selben Abstand X zur Flachseite 20 der zweiten Komponente angeordnet. Beide Punkte, Schnittpunkt M und Punkt A sind beabstandet zueinander angeordnet. Im Anschluss an den gekrümmten Flächenabschnitt 23 kann ein weiterer gekrümmter Flächenabschnitt 30 angeordnet sein. Die gekrümmt ausgebildeten Flächenabschnitte 23 und 30 können bezüglich ihrem Krümmungsgrad und ihrer Länge gleiche oder unterschiedlich große Werte umfassen. Der Flächenabschnitt 23 ist als Anstieg, der Flächenabschnitt 30 als Abstieg ausgebildet. Obige Beschreibung für den Anstieg kann auch für den Abstieg gelten, mit dem Unterschied, dass sich beim Abstieg der Durchmesser der Nut 10 sich vom Scheitelpunkt K in Richtung Boden 12 vergrößert. An- und Abstieg können auch unterschiedlich geformt sein. Die gekrümmt ausgebildeten Flächenabschnitte 23, und 30, und der Scheitelpunkt K, der zwischen diesen beiden Flächenabschnitten 23 und 30 liegt, bilden die Krümmung, die Kontaktfläche 29. Im Anschluss an den gekrümmten Flächenabschnitt 30 kann ein weiterer Flächenabschnitt 22, der gleich oder ähnlich dem Flächenabschnitt 21 ist, angeordnet und ausgebildet sein. Im Ausführungsbeispiel gemäß Figur 3 wird die Umfangsfläche 16 der Nut 10 durch wenigstens zwei, gezeigt sind vier (21, 23, 22, 30), Flächenabschnitte gebildet. Dabei entspricht der Kontaktbereich 29 den Flächenabschnitten 23 und 30, zwischen denen der Kontaktpunkt KP angeordnet ist. Die Flächenabschnitte 23, 30 weisen jeweils eine Krümmung auf, die der vorstehenden Beschreibung folgt. Der Anteil des Bereichs der Flächenabschnitte 21, 23 und 30, 23 können, bezogen auf die Umfangsfläche 16, gleich groß oder verschieden groß sein.

Diese Ausführungsform ist ein Beispiel dafür, dass die Kontaktfläche 29 nur einen Teil der Umfangsfläche 16 bildet. Nur dieser Bereich ist maßgeblich für die Festigkeit der Verbindung, denn nur in diesem Bereich liegt der Kontaktpunkt KP.

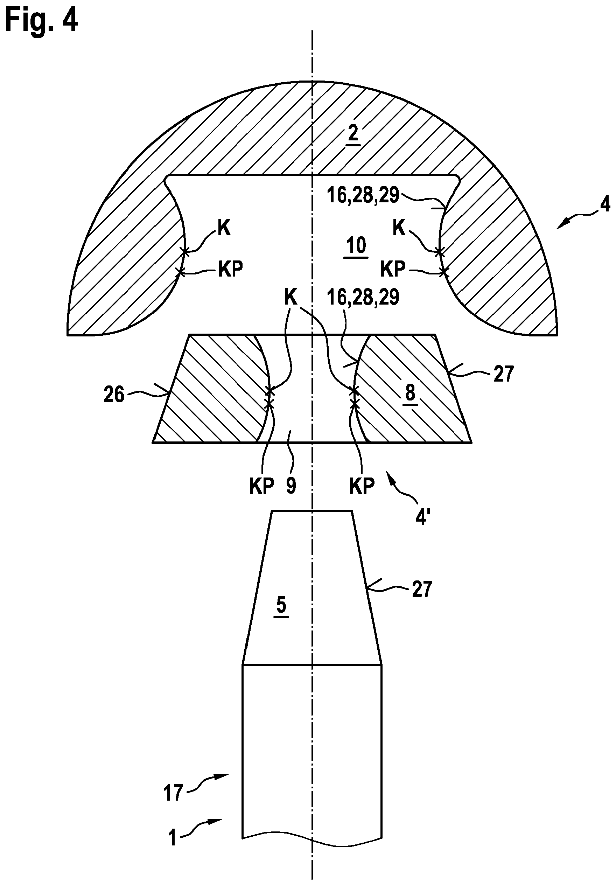

Figur 4 zeigt ein orthopädisches System bestehend aus einer ersten Komponente 1, einer zweiten Komponente 2 und einer dritten Komponente 8, einem Verbindungsteil, einem Adapter 8. Gleiche Bezugszeichen haben die gleiche Bedeutung wie vorstehend beschrieben. Dieses orthopädische System weist zwei nicht-erfindungsgemäße Verbindungsanordnungen 4 und 4' auf. Eine erste Verbindungsanordnung 4 ist zwischen der zweiten Komponente 2 und dem Adapter 8, und eine zweite Verbindungsanordnung 4' ist zwischen dem Adapter 8 und der ersten Komponente 1 angeordnet. Für die Verbindungsanordnung 4 zwischen der zweiten Komponente 2 und dem Adapter 8 gilt vorstehende Beschreibung.

Der Adapter 8 weist eine Nut 9 in Form einer Durchgangsöffnung auf. Die Kontaktfläche 29 an der Umfangsfläche 16, die Oberfläche 28 der Nut 9, ist entsprechend vorstehender Beschreibung gekrümmt ausgebildet. Der Schaft 17 der ersten Komponente 1 ist entsprechend vorstehender Beschreibung ausgebildet. Durch eine Anordnung gemäß Figur 4 kann ein orthopädisches System aus mehreren Komponenten 1, 2, 8 mit mehreren nicht-erfindungsgemäßen Verbindungsanordnungen 4, 4' bereitgestellt werden.

Alternativ zu der in Figur 4 gezeigten Anordnung der gekrümmt ausgebildeten Kontaktfläche 29 kann diese auch an anderen Flächen, beispielsweise an der Außenumfangsfläche 26 des Adapters 8 oder am Vorsatz 5 der ersten Komponente 1, angeordnet sein. Unabhängig von der Anordnung der gekrümmt ausgebildeten Kontaktfläche 29 weist eine nicht-erfindungsgemäße Verbindungsanordnung 4, 4' eine gekrümmte Fläche 16, 28, 29 angeordnet an einer Komponente und eine mit dieser in Wirkverbindung stehender Kontaktfläche 27 angeordnet an einer zweiten oder dritten Komponente auf. Dabei kann es sich sowohl bei der gekrümmten Fläche 29 wie auch bei der Kontaktfläche 27 um Teilbereiche einzelner Flächen handeln.

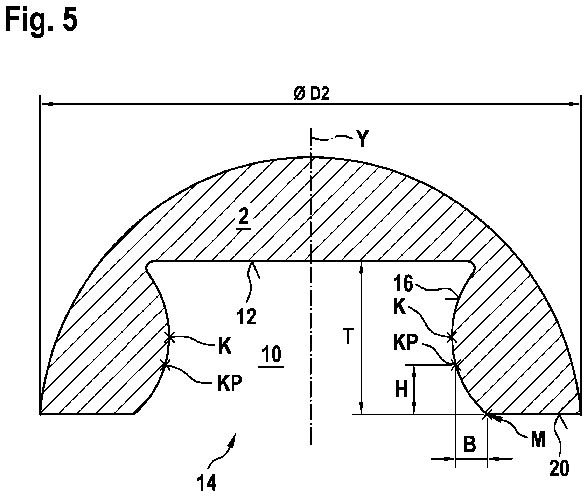

Figur 5 zeigt ein nicht-erfindungsgemäßes Beispiel einer Komponente 2 eines Kugelkopfes mit einem Durchmesser D2 und die Positionierung des Kontaktpunkts KP an der Umfangsfläche 16. Der Kontaktpunkt KP ist in einem Abstand H zur Flachseite 20 angeordnet. T ist die Tiefe der Nut 10 und erstreckt sich vom Boden 12 der Nut 10 bis zur Mündung 14 zur Flachseite 20 der Komponente 2. Die Umfangsfläche 16 der Nut 10 ist wie vorstehend gekrümmt ausgebildet. Ausgehend vom Schnittpunkt M, der gemäß Figur 5 an der Flachseite 20 angeordnet ist, erstreckt sich die Krümmung in Richtung der Mittelachse Y. In dessen Folge ist der Kontaktpunkt KP in Richtung der Mittelachse Y versetzt zum Schnittpunkt M angeordnet. Zwischen dem Kontaktpunkt KP und dem Schnittpunkt M ist ein Abstand B ausgebildet. Im Ausführungsbeispiel gemäß Figur 5 entspricht der Wert von H = T/3 Abhängig von den Anwendungen kann der Kontaktpunkt KP an anderer Stelle angeordnet sein, aber er ist immer unterhalb des Scheitelpunkts K angeordnet.

Eine nicht-erfindungsgemäße Verbindungsanordnung zweier Komponenten weist an wenigstens einer dieser Komponenten einen gekrümmten Flächenabschnitt mit einem Scheitelpunkt K und einem Kontaktpunkt KP auf. Der gekrümmte Flächenabschnitt kann sich über die gesamte Oberfläche beispielsweise einer Nut erstrecken. Dann entsteht aus den Scheitelpunkten ein geschlossener Scheitelkreis, eine Umfangslinie, die aus mehreren aneinandergereihten Kontaktpunkten KP gebildet ist. Nach dem Zusammenfügen beider Komponenten entsteht an diesem Scheitelpunkt, beziehungsweise an diesem Scheitelkreis, eine linienförmige Wirkverbindung.

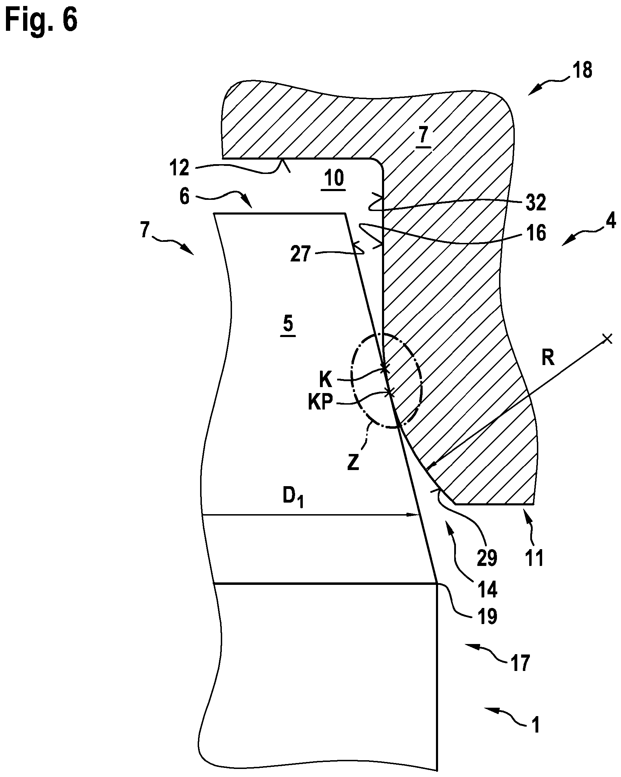

Figur 6 zeigt eine erfindungsgemäße Verbindungsanordnung 4 zweier orthopädischer Komponenten, hier eines Schafts 17 einer ersten Komponente 1, mit einem Kugelkopf 18 einer zweiten Komponente 2. Der Schaft 17 weist an seinem ersten Ende 7 einen Vorsprung oder Vorsatz 5 auf. Ausgehend von einer Stelle 19 des Schafts 17 verringert sich der Durchmesser D1 des Vorsatzes 5 bis zu dessen distalen Ende 6 kontinuierlich und bildet einen Kegelstumpf. Der Kegelstumpf weist eine Umfangs- bzw. Kontaktfläche 27 auf. Beide Komponenten können, wie auch alle anderen Ausführungsvarianten, Teil einer Hüftgelenkprothese, einer Schultergelenkprothese oder einer Finger- oder Fußgelenkprothese oder andere Gelenkprothesen sein.

Die zweite Komponente 2 weist eine Ausnehmung oder Nut 10 auf. Die Nut 10 wird durch ein Umfangsfläche 16 und einer Grundfläche beziehungsweise einem Boden 12 und gegenüber dem Boden von einer Mündung 14 begrenzt. Die Umfangsfläche 16 ist in dieser Ausführungsform zweiteilig ausgeführt, d.h. sie besteht aus zwei verschiedenen geometrischen Formen. Ausgehend vom zweiten Ende 11 der Komponente 2, von der Mündung 14 der Nut 10 ist die Umfangsfläche 16 der Nut 10 gekrümmt ausgebildet und bildet die Kontaktfläche 29, auf der sich der Kontaktpunkt KP befindet. Durch die Krümmung der Kontaktfläche 29 entsteht eine konvex gekrümmte Oberfläche. Ausgehend von der Mündung 14 der Nut 10 nimmt der Betrag des Durchmessers D1 der Nut 10 ab bis zu einem Punkt, dem Scheitelpunkt K. Ab diesem Scheitelpunkt K bleibt der Betrag des Durchmessers D1 bis zum Boden 12 konstant, d.h. ab diesem Scheitelpunkt K bildet die Umfangsfläche 16 einen Hohlzylinder 32. Der Hohlraum im Hohlzylinder 32 dient zur Aufnahme des restlichen Teils des Schafts 17, dem Teil des Vorsatzes 5 der zwischen dem Kontaktpunkt KP und dem distalen Ende 6 des Schafts 17 angeordnet ist. In diesem Bereich wird sich im implantierten Zustand Gelenkflüssigkeit befinden.

Die Kontaktfläche 29 ist kreisförmig ausgebildet.

Beim Zusammenfügen des Schafts 17 der ersten Komponente 1 mit dem Kugelkopf 18 der zweiten Komponente 2 entsteht an dem Kontaktpunkt KP der Kontakt beider Komponenten. Der Kontaktpunkt KP liegt auf der Umfangsfläche 16 bzw. der Kontaktfläche 29 unterhalb des Scheitelpunkts K in Richtung zum zweiten Ende 11 bzw. zur Mündung 14. Die Entfernung des Kontaktpunktes KP vom Scheitelpunkt K ist abhängig von der geometrischen Ausbildung des Kegelstumpfes der ersten Komponente 1 und der Krümmung der Kontaktfläche 29 der zweiten Komponente 2.

Diese erfindungsgemäße Ausführungsform hat den entscheidenden Vorteil, dass sich durch die kreisförmige Ausbildung der Kontaktfläche 29 die genaue örtliche Lage des Kontaktpunktes KP leicht, u.a. aus dem Konuswinkel des Vorsprungs 5 und dem Radius R, bestimmen lässt. Die Ausbildung des Hohlraums oberhalb des Scheitelpunkts K als Hohlzylinder 32 erleichtert die Herstellung der Komponente 2 erheblich.

Figur 7 zeigt einen Ausschnitt Z aus Figur 6. Es ist die kreisförmige Kontaktfläche 29 zu sehen, auf der sich der Kontaktpunkt KP befindet und die am Scheitelpunkt K in den Hohlzylinder 32 übergeht. Am Kontaktpunkt KP berührt die Kontaktfläche 29 die Kontaktfläche 27 des Vorsprungs 5 des Schafts 17.

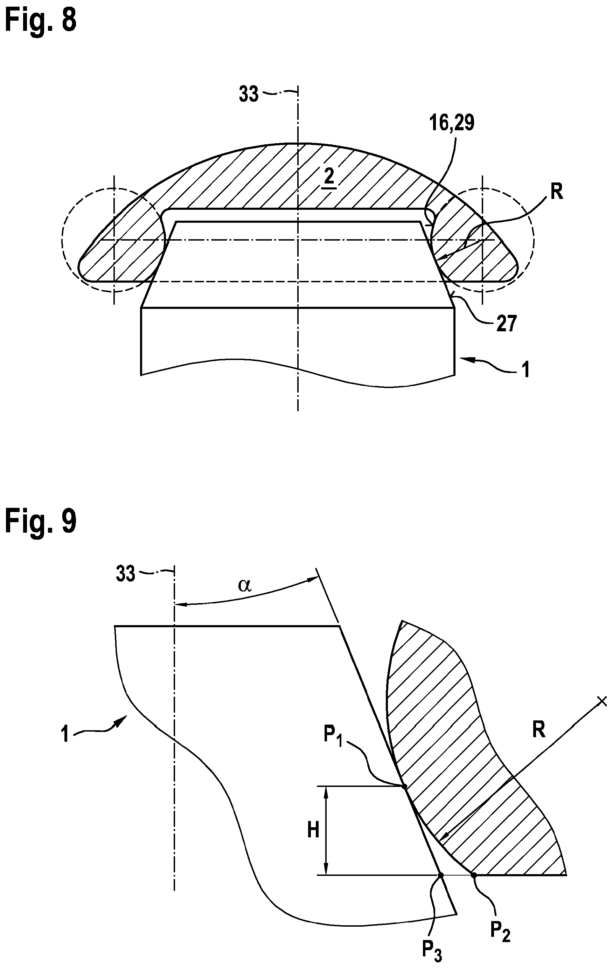

Figur 8 zeigt noch einmal schematisch eine nicht-erfindungsgemäße Verbindungsanordnung 4 mit einer zweiten orthopädischen Komponente 2, nämlich einem Kugelkopf und einer ersten orthopädischen Komponente, nämlich einem Schaft 1, dessen oberes Ende als Konus ausgebildet ist. Die Umfangsfläche 16 bzw. die Kontaktfläche 29 des Kugelkopfes ist als Teil eines Ringes mit dem Radius R ausgebildet. Zur Verdeutlichung ist ein kompletter Ring eingezeichnet. Die Längsachse ist mit dem Bezugszeichen 33 gekennzeichnet. In dieser Ausführungsform bildet die Längsachse 33 auch zugleich die Rotationsachse.

Figur 9 zeigt die Verbindungsstelle, d.h. einen Kontaktpunkt KP gemäß Figur 8 vergrößert, auszugsweise und in schematischer Darstellung. Am Kontaktpunkt KP, hier mit P1 bezeichnet, berührt der als Konus ausgebildete Teil der ersten orthopädischen Komponente 1 den als Ring mit dem Radius R ausgebildeten Kontaktbereich 29 des Kugelkopfes. Der Konuswinkel des Vorsprungs 5 ist hier mit α gekennzeichnet und bildet den "male taper". Der Abstand P2-P3 bezeichnet den Spalt an der Öffnung, d.h. den Öffnungsspalt (Gap at Orifice). Der Kontaktpunkt P1 befindet sich in einer Höhe H vom Öffnungsspalt entfernt.

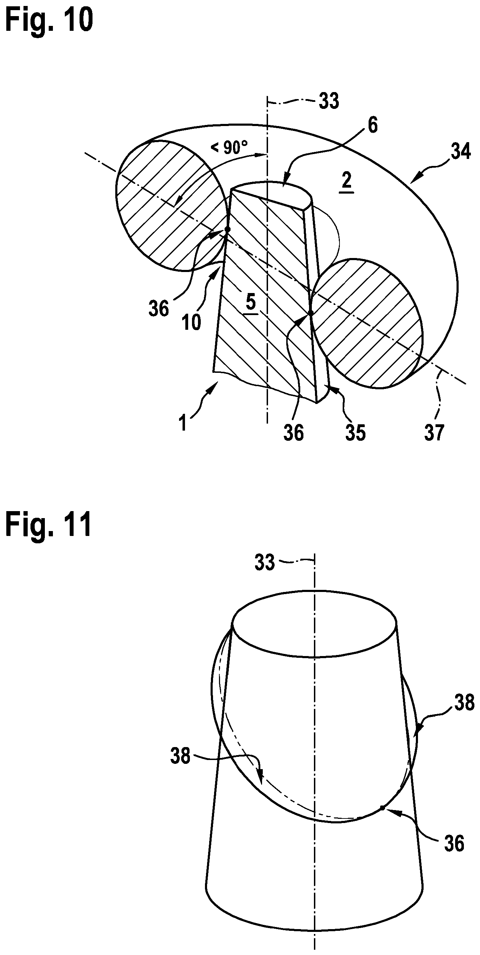

In den Figuren 10 und 11 ist in einer 3D Darstellung der Fügevorgang zweier Komponenten 1, 2 dargestellt, die zunächst in einem Winkel, der von einer orthogonalen Ausrichtung abweicht, angeordnet sind. Der Vorsatz 5 einer ersten Komponente 1 wird in eine Nut 10 einer zweiten Komponente 2 eingeführt, wobei zunächst ein 2-Punktkontakt zwischen dem Vorsatz 5, dem Konus 35 einer ersten Komponente1 und einer ringförmigen Kontaktfläche 29 einer zweiten Komponente 2 entsteht. Dieser 2-Punktkontakt entsteht dann, wenn zwei Komponenten (1 und 2), in einem Winkel, der von 90° abweicht, zueinander angeordnet sind. Bezugszeichen 33 zeigt die Längsachse von Komponente 1, Bezugszeichen 37 zeigt die Längsachse von Komponente 2. Diese beiden Achsen sind in einem Winkel, der von 90° abweicht, zueinander angeordnet. Daraus ergibt sich dann zunächst ein 2-Punktkontakt. Mit dem Bezugszeichen 36 sind die Kontaktpunkte eines 2-Punktkontakts bezeichnet (siehe Figur 10).

Figur 11 zeigt den Konus 35 gemäß Figur 10 sowie die Kontaktfläche 29 der Komponente 2. Komponente 1 und 2 sind in einem Winkel, abweichend von 90°, zueinander angeordnet. Dadurch einsteht eine 2-Kontaktpunkt Verbindung zwischen der Komponente 1 und 2. Einer der beiden Kontaktpunkte ist gezeigt und mit dem Bezugszeichen 36 versehen. Der zweite Kontaktpunkt ist gegenüber dem ersten Kontaktpunkt in einem Abstand von 180° angeordnet. Zwischen den Kontaktpunkten besteht ein Spalt 38, der sich ausgehend von einem Kontaktpunkt 36 in Richtung des zweiten Kontaktpunktes 36 vergrößert. Der Spalt 38 weist seine größte Weite im Bereich der Hälfte des Abstands der Kontaktpunkte auf.

Der Fügezustand gemäß den Figuren 10 und 11 zweier Komponenten ist nicht stabil und dauerhaft und zu vermeiden. Die erfindungsgemäße gewölbte Oberfläche an wenigstens einer Komponente überführt die Verbindung beim weiteren Zusammenfügen von einer 2-Kontaktpunkt Verbindung zu einem Linienkontakt. Dabei wird, ausgehend vom Fügezustand gemäß Figur 10, beispielsweise der Vorsatz 5 der ersten Komponente 1 weiter in die Nut 10 der zweiten Komponente 2 eingeführt. Die erfindungsgemäß gewölbt ausgebildete Kontaktfläche 29 überführt die 2-Kontaktpunkt Verbindung zu einem Linienkontakt und zugleich zu einer orthogonalen Ausrichtung beider Komponenten 1 und 2. Dadurch ist es möglich den Fügezustand gemäß Figuren 10 und 11 in einen Fügezustand gemäß Figur 1 zu überführen. Im Ausführungsbeispiel gemäß Figur 1 sind die beiden Komponenten 1 und 2 im 90°-Winkel zueinander angeordnet. Dadurch entsteht ein Linienkontakt, der sich über die gesamte Umfangsfläche erstreckt.

## Patentansprüche

1. Verbindungsanordnung (4, 4') eines orthopädischen Systems zum Verbinden mehrerer Komponenten (1, 2, 8), wobei die Verbindungsanordnung (4, 4') eine erste Komponente (1) und eine zweite Komponente (2) umfasst,
wobei die erste Komponente (1) an einem ersten Ende einen Vorsatz aufweist, wobei der Vorsatz in Form eines Kegelstumpfes ausgebildet ist, dessen Durchmesser ausgehend vom distalen Ende zunimmt,
wobei die erste Komponente (1) mit wenigstens einer Kontaktfläche (27) ausgebildet ist, und wobei die Kontaktfläche (27) der ersten Komponente (1) als Konus ausgebildet ist,
wobei die zweite Komponente (2) an einem zweiten Ende eine Ausnehmung in Form einer Nut (10) aufweist,
wobei die Nut (10) durch einen Boden (12), eine Umfangsfläche (16) mit einer gewölbten, gekrümmten Oberfläche und eine Mündung (14) gebildet ist,
wobei die Umfangsfläche (16) der Nut (10) ganz oder teilweise als Kontaktfläche (29) der zweiten Komponente (2) ausgebildet ist,
so dass die Kontaktfläche (29) der zweiten Komponente (2) gekrümmt geformt ist,
wobei die zweite Komponente (2) der Kugelkopf (18) einer Hüftgelenkprothese, einer Schultergelenkprothese oder einer Finger- oder Fußgelenkprothese oder andere Gelenkprothese ist und die erste Komponente (1) der Schaft dieser Prothese ist,
**dadurch gekennzeichnet,**
**dass** die Kontaktfläche (29) der zweiten Komponente (2) im Querschnitt konvex geformt ist, einen kreisförmigen Querschnitt aufweist und der Querschnitt sich über die gesamte Kontaktfläche (29) erstreckt, und
**dass** ausgehend vom distalen Ende der Nut (10) der Querschnitt der Nut über einen ersten Bereich abnimmt, bis der kleinste Querschnitt als Scheitelpunkt K erreicht ist, und
**dass** der Querschnitt der Nut (10) ab dem Scheitelpunkt (K) bis zum Boden (12) der Nut (10) konstant bleibt,
wobei die Umfangsfläche (16) ab dem Scheitelpunkt (K) einen Hohlzylinder (32) ausbildet, und
**dass** die Kontaktfläche (27) der ersten Komponente (1) und die Kontaktfläche (29) der zweiten Komponente im Querschnitt gesehen einen Kontaktpunkt (KP) aufweisen und die Kontaktfläche (27) der ersten Komponente (1) mit der Kontaktfläche (29) der zweiten Komponente (2) im Bereich des Kontaktpunktes (KP) in Wirkverbindung steht, wobei sich die Wirkverbindung entlang wenigstens einer Umfangslinie, die durch eine Vielzahl aneinander gereihter Kontaktpunkte (KP) gebildet ist, erstreckt, und
**dass** der Kontaktpunkt (KP) auf der Kontaktfläche (27) der ersten Komponente (1) und der Kontaktfläche (29) der zweiten Komponente (2) unterhalb des Scheitelpunktes (K) in Richtung zur Mündung (14) liegt.

2. Verbindungsanordnung nach Anspruch 1, **dadurch gekennzeichnet, dass** der Kurvenverlauf der Kontaktfläche (29) stetig ausgebildet ist, wobei deren Funktion an jedem Punkt zweifach stetig differenzierbar ist.

## Claims

1. A connection assembly (4, 4') of an orthopedic system for connecting a plurality of components (1, 2, 8), the connection assembly (4, 4') comprising a first component (1) and a second component (2),
wherein the first component (1) includes an attachment at a first end, wherein the attachment is configured in the form of a truncated cone whose diameter increases proceeding from the distal end,
wherein the first component (1) is formed with at least one contact surface (27), and wherein the contact surface (27) of the first component (1) is designed as a cone,
wherein at a second end the second component (2) includes a recess in the form of a groove (10),
wherein the groove (10) is formed by a base (12), a peripheral surface (16) with a convex, curved surface and a mouth (14),
wherein the peripheral surface (16) of the groove (10) is wholly or partly formed as a contact surface (29) of the second component (2),
so that the contact surface (29) of the second component (2) is of curved shape,
wherein the second component (2) is the ball head (18) of a hip joint prosthesis, a shoulder joint prosthesis or a finger or foot joint prosthesis or another joint prosthesis, and the first component (1) is the stem of this prosthesis,
**characterized in**
**that** the contact surface (29) of the second component (2) is convex in cross-section, has a circular cross-section, and the cross-section extends over the entire contact surface (29), and
**that** proceeding from the distal end of the groove (10) the cross-section of the groove decreases over a first region, until the smallest cross-section is reached as the apex (K), and
**that** the cross-section of the groove (10) remains constant from the apex (K) to the base (12) of the groove (10),
wherein the peripheral surface (16) forms a hollow cylinder (32) from the apex (K), and
**that** the contact surface (27) of the first component (1) and the contact surface (29) of the second component have a contact point (KP) when viewed in cross-section, and the contact surface (27) of the first component (1) is operatively connected to the contact surface (29) of the second component (2) in the region of the contact point (KP), wherein the operative connection extends along at least one peripheral line which is formed by a plurality of contact points (KP) arranged in a row, and
**that** the contact point (KP) lies on the contact surface (27) of the first component (1) and on the contact surface (29) of the second component (2) below the apex (K) in the direction towards the mouth (14).

2. The connection assembly according to claim 1, **characterized in that** the curve profile of the contact surface (29) is continuous, it being possible to continuously differentiate its function twice at any point.

## Revendications

1. Agencement de raccordement (4, 4') d'un système orthopédique pour le raccordement de plusieurs composants (1, 2, 8), l'agencement de raccordement (4, 4') comprenant un premier composant (1) et un deuxième composant (2),
dans lequel le premier composant (1) présente à une première extrémité une protubérance, la protubérance étant réalisée sous forme d'un cône tronqué dont le diamètre augmente à partir de l'extrémité distale,
dans lequel le premier composant (1) est réalisé avec au moins une surface de contact (27), et dans lequel la surface de contact (27) du premier composant (1) est réalisée sous forme de cône,
dans lequel le deuxième composant (2) présente à une deuxième extrémité un évidement sous forme d'une rainure (10),
dans lequel la rainure (10) est formée par un fond (12), une surface circonférentielle (16) ayant une surface incurvée bombée, et une embouchure (14),
dans lequel la surface circonférentielle (16) de la rainure (10) est réalisée entièrement ou partiellement comme surface de contact (29) du deuxième composant (2),
de sorte que la surface de contact (29) du deuxième composant (2) est de forme incurvée,
dans lequel le deuxième composant (2) est la tête sphérique (18) d'une prothèse d'articulation de la hanche, d'une prothèse d'articulation de l'épaule ou d'une prothèse d'articulation de doigt ou de pied ou d'une autre prothèse d'articulation et le premier composant (1) est la tige de ladite prothèse,
**caractérisé en ce que**
la surface de contact (29) du deuxième composant (2) est de forme convexe en section transversale, présente une section transversale circulaire et la section transversale s'étend sur toute la surface de contact (29), et
partant de l'extrémité distale de la rainure (10), la section transversale de la rainure diminue sur une première plage jusqu'à ce que la section transversale la plus petite soit atteinte en tant que sommet K, et
la section transversale de la rainure (10) reste constante à partir du sommet (K) jusqu'au fond (12) de la rainure (10),
la surface circonférentielle (16) forme un cylindre creux (32) à partir du sommet (K), et
la surface de contact (27) du premier composant (1) et la surface de contact (29) du deuxième composant présentent, vu en section transversale, un point de contact (KP) et la surface de contact (27) du premier composant (1) est en liaison active avec la surface de contact (29) du deuxième composant (2) dans la zone du point de contact (KP), la liaison active s'étendant le long d'au moins une ligne circonférentielle qui est formée par une pluralité de points de contact (KP) juxtaposés, et
le point de contact (KP) sur la surface de contact (27) du premier composant (1) et sur la surface de contact (29) du deuxième composant (2) se trouve en dessous du sommet (K) en direction de l'embouchure (14).

2. Agencement de raccordement selon la revendication 1, **caractérisé en ce que** le tracé de courbe de la surface de contact (29) est continu, la fonction de celui-ci étant doublement différentiable en continu à chaque point.
